(19) Europäisches Patentamt
European Patent Office
Office européen des brevets

(11) **EP 3 073 912 B1**

(12) **EUROPÄISCHE PATENTSCHRIFT**

(45) Veröffentlichungstag und Bekanntmachung des Hinweises auf die Patenterteilung:
**31.01.2018 Patentblatt 2018/05**

(21) Anmeldenummer: **13802304.9**

(22) Anmeldetag: **29.11.2013**

(51) Int Cl.:
***A61B 5/05*** (2006.01)

(86) Internationale Anmeldenummer:
**PCT/EP2013/075148**

(87) Internationale Veröffentlichungsnummer:
**WO 2015/078527 (04.06.2015 Gazette 2015/22)**

(54) **VERFAHREN ZUM MAGNETIC PARTICLE IMAGING MIT UNBESCHRÄNKTEM AXIALEN FIELD OF VIEW**

METHOD FOR MAGNETIC PARTICLE IMAGING WITH AN UNRESTRICTED AXIAL FIELD OF VIEW

PROCÉDÉ D'IMAGERIE À PARTICULES MAGNÉTIQUES À CHAMP DE VISION AXIAL ILLIMITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.10.2016 Patentblatt 2016/40**

(73) Patentinhaber: **Universität zu Lübeck**
**23538 Lübeck (DE)**

(72) Erfinder:
• **BUZUG, Thorsten**
  **23627 Groß Sarau (DE)**
• **KAETHNER, Christian**
  **23522 Lübeck (DE)**
• **AHLBORG, Mandy**
  **23564 Lübeck (DE)**
• **BRINGOUT, Gael**
  **23552 Lübeck (DE)**
• **WEBER, Matthias**
  **23564 Lübeck (DE)**

(74) Vertreter: **Schmelcher, Thilo**
**RCD-Patent**
**Postfach 3106**
**52118 Herzogenrath (DE)**

(56) Entgegenhaltungen:
**WO-A1-2011/030249**

**Beschreibung**

Die Erfindung betrifft ein Verfahren zur tomographischen Bildgebung eines Objekts mit im Objektinnern verteilten magnetischen Partikeln.

Beim Verfahren des Magnetic Particle Imaging (MPI) werden lokale Konzentrationen magnetisierbarer Nanopartikel in einer a priori unbekannten räumlichen Verteilung im Innern eines Objekts ermittelt. Beispielsweise können super-paramagnetische Eisenoxidpartikel in einer Untersuchungsregion durch ein mit vorbestimmter Frequenz periodisch veränderliches Magnetfeld, nachfolgend auch als Anregungsfeld (engl. "drive field") bezeichnet, periodisch magnetisiert werden, wobei die Magnetisierung der Partikel nichtlinear von der Gesamtmagnetfeldstärke abhängt. Erfasst und analysiert man das Zeitverhalten der Partikelmagnetisierung z.B. mit Hilfe von Detektionsspulen, so weist das Messsignal höhere Harmonische der Frequenz des Anregungsfeldes auf, und aus einer Fourier-Analyse des Signals kann auf die Partikelkonzentration geschlossen werden. Alternativ hierzu kann die Bildrekonstruktion auch mit dem x-Space-Verfahren erfolgen.

Zur Eingrenzung kleiner Volumina der Untersuchungsregion wird das Anregungsfeld mit einem - üblicherweise zeitlich konstanten - Selektionsmagnetfeld (engl. "selection field") überlagert. Das Selektionsmagnetfeld weist an wenigstens einem vorbestimmten Punkt der Untersuchungsregion eine Nullstelle auf. Ausgehend von diesem so genannten feldfreien Punkt, kurz: FFP, steigt das Selektionsmagnetfeld in alle Richtungen schnell an, so dass magnetisierbare Nanopartikel schon in geringem Abstand zum FFP in die magnetische Sättigung gelangen. Weit vom FFP entfernte Nanopartikel reagieren dann kaum noch auf das Anregungsfeld und leisten keinen signifikanten Beitrag zum detektierten Signal. Das MPI-Signal stammt vielmehr aus der lokalen Umgebung des FFP und gibt über die dort vorhandene lokale Partikelkonzentration Auskunft.

Mit Hilfe des Anregungsfeldes wird der FFP innerhalb der Untersuchungsregion verschoben; der FFP durchläuft dabei eine Bahnkurve, nachfolgend auch als Trajektorie bezeichnet, die offen sein kann, in der Regel jedoch geschlossen ist, so dass der FFP nach einer vorbestimmten Repetitionszeit an seinen Ausgangspunkt zurückkehrt. Das Anregungsfeld ist zeitabhängig und wird üblicherweise von Elektromagneten erzeugt.

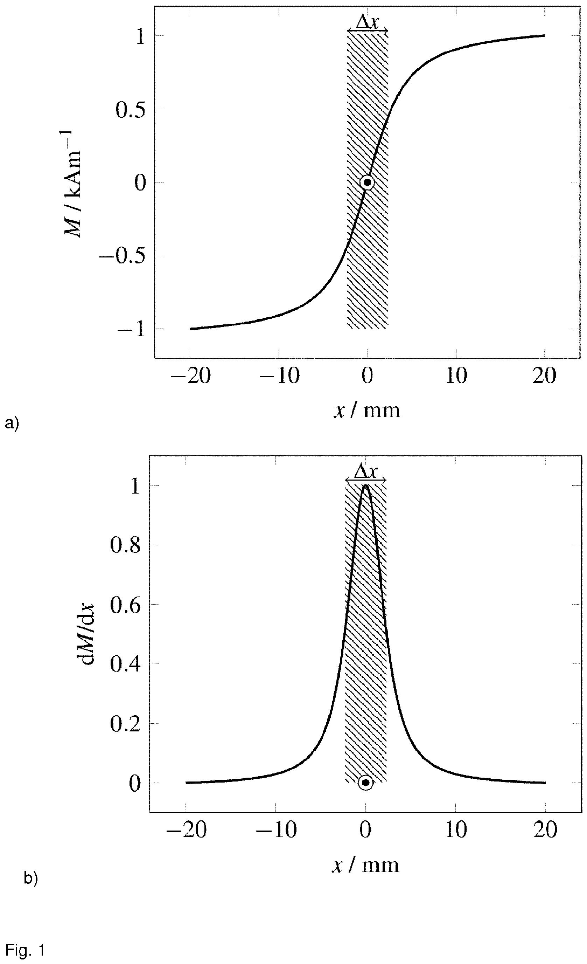

In Fig. 1 a) ist ein typischer Magnetisierungsverlauf von super-paramagnetischen Eisenoxid-Partikeln dargestellt. Unter der Wirkung eines Selektionsmagnetfeldes, welches bei x = 0 einen FFP mit einem Magnetfeldgradienten G einrichtet, zeigt die Magnetisierung eines Partikels näherungsweise den Verlauf einer Langevin-Funktion. Nur innerhalb einer Umgebung der Breite $\Delta x$ um den FFP befindet es sich nicht in der magnetischen Sättigung und antwortet auf das Anregungsfeld des MPI. Üblicherweise wird die Breite $\Delta x$ mit der Peakbreite der Ableitung dM/dx gleichgesetzt, die wiederum als Abstand der beiden Werte dM/dx = ½ (engl. "full width half maximum", FWHM) definiert ist, wie gezeichnet in Fig. 1 b). Die Ableitung der Magnetisierungskurve wird auch als Punktspreizfunktion (engl. "point spread function") bezeichnet. Ein punktförmiges Partikel ist mit einer MPI-Messung prinzipiell nur bis auf ein Intervall genau lokalisierbar.

Wie man anhand von Fig. 1a) und Fig. 1b) erkennt, zieht ein höherer Magnetfeldgradient G im FFP auch einen steileren Verlauf der Langevin-Funktion durch x = 0 und somit einen schmaleren Peak nach sich. Da die Langevin-Funktion um x = 0 in führender Ordnung linear ist, verhalten sich $\Delta x$ und G dort zueinander antiproportional. Die Proportionalitätskonstante ist eine Eigenschaft der verwendeten Partikel, die so genannte magnetische Wirksamkeit $X_S$.

$$\Delta x = X_S / G \qquad\qquad\qquad (1)$$

Die magnetische Wirksamkeit eines Partikeltyps ist üblicherweise vorbekannt. Sie kann ansonsten auch einfach durch Vorversuche bestimmt werden. Ein gängiger Wert für das Kontrastmittel Resovist ist z.B. $X_S = 3 * 10^{-3}$ T, d.h. bei einem Magnetfeldgradienten von G = 1 T/m beträgt die Breite der Punktspreizfunktion etwa 3 Millimeter.

Unter dem "Field of View" (FOV) einer MPI-Messvorrichtung, nachfolgend auch als MPI-Scanner bezeichnet, versteht man den Raumbereich, den ein FFP während des Verlaufs der Messung überstreichen kann. Das FOV kann kleiner oder größer als das Volumen eines zu messenden Objekts sein. Beim Messvorgang wird die Änderung des Magnetisierungszustandes der im Objekt verteilten magnetischen Partikel erfasst und fortlaufend mit der aktuellen Position des FFP registriert. Dabei wird nicht jeder Raumpunkt des FOV zwangsläufig von einem FFP durchlaufen, aber idealerweise kommt ein FFP jedem der Raumpunkte irgendwann nahe genug, dass auch Partikelkonzentrationen aus den nicht durchlaufenen Raumpunkten Signalbeiträge liefern.

In der Praxis unterteilt man das FOV daher gewöhnlich in würfelförmige Voxel mit der Kantenlänge $\Delta x$ (wie oben definiert) oder kleiner und zielt darauf ab, für möglichst jeden Voxel im Objektinnern wenigstens einen MPI-Messwert zu erfassen. Ein derart abgetastetes FOV ist dann ohne Probleme - typisch linear - interpolierbar, d.h. zwischen je zwei Raumpunkten, zu denen mittels MPI ermittelte Konzentrationswerte vorliegen, können auf dazwischen liegenden Raumpunkten Konzentrationswerte mit einer abstandsabhängigen Gewichtung verlässlich geschätzt werden. Das vorbeschriebene Vorgehen zur MPI-Messwerterfassung in nahezu jedem Voxel soll im Folgenden auch als "ausreichend dichte" Abtastung des FOV bezeichnet werden.

Unter anderem aus der US 2012/153949 A1 ist bekannt, dass sich Lissajous-Trajektorien mittels zweier harmonisch zeitabhängiger, homogener Magnetfelder entlang orthogonaler Achsen (z.B. x- und y-Achse) generieren lassen, wobei die bevorzugten Trajektorien für MPI zahlreiche Kreuzungspunkte aufweisen und beispielsweise der Bedingung

$$fy \: / \: fx = Nd \: / \: (Nd + 1) \tag{2}$$

genügen. Dabei bezeichnen fx und fy die Frequenzen entlang der x- und y-Achse, respektive. Nd ist eine natürliche Zahl, und die Zahl der Kreuzungspunkte der Trajektorie beträgt dann ungefähr $Nd * Nd$. Ab einem Frequenzverhältnis $fy \: / \: fx > 0{,}98$ spricht man von einer "high density" Trajektorie.

Das FOV eines MPI-Scanners mit einer solchen Trajektorie ist im Wesentlichen zweidimensional - genau genommen wird eine Schicht der Dicke $\Delta x$ gescannt - und typisch von etwa quadratischer Form. Der FFP bewegt sich in einer Ebene, die nachfolgend auch als Scanebene bezeichnet wird. Der Durchmesser des FOV, d.h. der maximale Abstand zweier vom FFP durchlaufenen Raumpunkte auf der Trajektorie, ist kleiner als der Abstand der Spulen, die die Anregungsfelder erzeugen, und kann insbesondere auch sehr viel kleiner eingerichtet werden.

Zur ausreichend dichten Abtastung des zweidimensionalen FOV eines MPI-Scanners, der eine Lissajous-Trajektorie mit Kreuzungspunkten und einem vorbestimmten Durchmesser erzeugt, muss man die magnetische Wirksamkeit der magnetisierbaren Partikel kennen und den Magnetfeldgradienten im FFP so wählen, dass die Breite $\Delta x$ der Punktspreizfunktion mit dem Abstand benachbarter Linien der Trajektorie bzw. mit dem Abstand benachbarter Kreuzungspunkte in etwa übereinstimmt.

Man kann einen MPI-Scanner auch mit einem dreidimensionalen FOV versehen, indem man ein weiteres Spulenpaar vorsieht und zu einer dreidimensionalen Trajektorie übergeht.

Aus der Arbeit von Borgert J., Gleich B., Rahmer J., Dahnke H., Weizenecker J., "Three-Dimensional Real-Time In Vivo Magnetic Particle Imaging", MedicaMundi, 53/2, 2009, S. 48-57 geht ein MPI-Scanner hervor, der Spulen zur Erzeugung und Überlagerung dreier senkrecht zueinander gerichteter, homogener Magnetfelder aufweist, mit denen der FFP innerhalb eines dreidimensionalen FOV beliebig positioniert werden kann. Damit ist ein MPI-Volumenscan mit einer vorbestimmten Messpunktdichte durchführbar. Insbesondere wird vorgeschlagen, den FFP entlang einer dreidimensionalen Lissajous-Figur als Trajektorie zu bewegen, indem jeder homogenen Magnetfeldkomponente eine harmonische Oszillation aufgeprägt wird und die drei Frequenzen kommensurabel eingerichtet werden. Die bislang so erzielbaren Abmessungen des FOV sind jedoch relativ klein; sie betragen nur einige Zentimeter.

Alternativ kann ein dreidimensionales FOV realisiert werden, indem an einem MPI-Scanner eine Einrichtung zum Verschieben des Objekts durch eine wie oben beschriebene zweidimensionale Scanebene vorgesehen wird. Beispielsweise verwendet man einen mittels elektrischer Antriebe verschiebbaren Tisch, auf dem das aufliegende Objekt durch eine Magnetfeld erzeugenden Spulenanordnung bewegt wird. In diesem Fall wird das ursprünglich zweidimensionale FOV des MPI-Scanners entlang der Vorschubrichtung expandiert, wobei es zweckmäßig ist, die Registrierung der MPI-Messdaten in einem fest mit dem Objekt verbundenen Koordinatensystem, nachfolgend auch als Objektkoordinaten bezeichnet, vorzunehmen.

Aus der Arbeit von Goodwill, P.W., Konkle, J.J., Zheng, B., Saritas, E.U., Conolly, S.M., "Projection X-Space Magnetic Particle Imaging", IEEE Trans. Med. Imag., 31 (5), 2012 ist ein MPI-Scanner bekannt, der dazu ausgebildet ist, feldfreie Linien, kurz: FFL, mittels Quadrupol-Selektionsmagnetfeldern zu generieren. Eine FFL kann als eine Aneinanderreihung einer Vielzahl von FFPs entlang einer Linie aufgefasst werden. Sie lässt sich analog zu FFPs mittels homogener Anregungsmagnetfelder verschieben, aber auch durch Variation des Quadrupol-Magnetfeldes rotieren, z.B. um einen Drehpunkt in der Mitte der Linie. Darüber hinaus - wie auch erwähnt in der WO 2010/008478 A2 - kommt die mechanische Verschiebung der FFL durch das zu vermessende Objekt in Betracht. In der genannten Arbeit von Goodwill et al. (2012) wird der Patiententisch (hier: Patient = vor der Messung getötete Maus) mechanisch entlang einer Achse durch die felderzeugende Spulenanordnung mit konstanter Geschwindigkeit, 2,5 cm/s, bewegt, während die FFL in einer Scanebene senkrecht zur Tischvorschubrichtung periodisch durch Magnetfeldmodulation hin und her bewegt wird. Auch diese Messanordnung besitzt senkrecht zur Vorschubrichtung ein FOV mit Abmessungen von nur wenigen Zentimetern, während das FOV entlang der Tischvorschubrichtung im Prinzip beliebig weit expandiert werden kann.

Zur tomographischen Bildgebung eines Objekts, das durch eine Scanebene hindurch bewegt wird, kann man das in Objektkoordinaten dreidimensionale FOV ebenfalls in typischerweise kubische Voxel mit Kantenlänge $\Delta x$ oder kleiner unterteilen und anstreben, jedem Voxel wenigstens einen MPI-Messwert zuzuordnen. Dabei wird man naheliegend das Objekt kontinuierlich verschieben und die Messwerte Schicht für Schicht erfassen, bis der interessierende Objektbereich die Scanebene passiert hat.

Die zum Scannen verwendeten Frequenzen der Anregungsfelder bei den oben beschriebenen MPI-Anordnungen mit dreidimensionalem FOV liegen jeweils oberhalb von 20 kHz. Die Repetitionszeiten der Trajektorien liegen in der Größenordnung Millisekunden, und man kann binnen weniger Minuten eine ausreichend dichte Abtastung des FOV erreichen.

Dies gilt erst recht bei Verwendung einer FFL anstelle eines einzelnen FFP. Doch wenngleich eine FFL zum Abtasten eines großen FOV auf den ersten Blick besonders geeignet erscheint, weil sie den Scanbedarf entlang einer der drei Raumdimensionen entfallen lässt, muss es doch als ein Nachteil angesehen werden, dass man als originäre MPI-Messdaten nur Signale erhält, die sich als Summen aller Partikelantworten entlang der FFL ergeben. Die rechnerbasierte Rekonstruktion lokaler Partikelkonzentrationen und deren Zuweisung auf Voxel innerhalb der FFL kann nur durch eine Auswertung erreicht werden, die der Bildrekonstruktion von Computertomographie (CT)-Bildern ähnlich ist. Naheliegend kann man erwarten, dass in jeder Schicht des Objekts Messdaten zu einer Mehrzahl unterschiedlich orientierter FFLs erfasst werden müssen, damit sich Partikelkonzentrationen für einzelne Voxel der Schicht ermitteln lassen.

In einer zukünftigen medizinischen Anwendung des MPI-Verfahrens wird man zur tomographischen Bildgebung beliebiger Körperteile oder auch des ganzen Körpers eines Patienten deutlich größere Spulenabstände realisieren müssen. Beispielsweise kann man davon ausgehen, dass die elektromagnetischen Spulen einen Abstand von etwa 0,5 m aufweisen müssen. Es sind dann relativ große Selektions- und Anregungsfeldamplituden zu erzeugen, damit über das gesamte FOV hinweg Magnetfeldgradienten der Größenordnung bis zu 1 T/m eingerichtet werden können. Der Patient wird während des Scanvorgangs insofern kontinuierliche Magnetfeldänderungen um die Größenordnungen 0,1 bis 1 T in seinem Körper erdulden müssen. Dies kann unerwünschte Nebenwirkungen haben.

Beispielsweise ist aus Reilly, J.P., "Magnetic field excitation of peripheral nerves and the heart: a comparison of thresholds", Med. Bio. Engin. Comp., 29 (6), 571-579, 1991 bekannt, dass zeitlich veränderliche Magnetfelder zu wahrnehmbaren - vereinzelt auch schmerzhaften - peripheren Nervenstimulationen (PNS) führen können. Verantwortlich dafür ist die Induktion elektrischer Feldgradienten im Bereich der Nervenfasern durch magnetische Flussdichteänderungen dB/dt der Größenordnung 100 T/s oder höher. Reilly gibt insbesondere Schwellenwerte zum Auslösen der PNS für die Flussdichteamplituden sinusförmig oszillierender Magnetfelder an, die ihrerseits von der Oszillationsfrequenz abhängen. Der Amplitudenbereich 0,1 bis 1 T beschränkt in einem humanen MPI-Scanner den sicheren - d.h. zuverlässig PNS-freien - Bereich hiernach auf Feldfrequenzen um 100 Hz, auf jeden Fall aber auf den Subkilohertzbereich.

Während die vorbeschriebenen MPI-Scanner jeweils ein FOV mit einem Volumen deutlich kleiner als 1 dm$^3$ aufweisen, müssen zukünftige medizinische MPI-Scanner mit einem FOV-Volumen im Bereich 10 bis 100 dm$^3$ oder mehr ausgestattet sein. Zudem können sie in einem humanen Ganzkörper-Scanner voraussichtlich nur periodische Anregungsfelder verwenden, die Frequenzanteile von maximal 1000 Hz, wahrscheinlich sogar nur wenige 100 Hz, aufweisen. Beides zusammen wird aber auf eine deutliche Erhöhung der Repetitionszeiten von FFP- oder FFL-Trajektorien führen und somit erzwingen, dass eine ausreichend dichte Abtastung des FOV für ein - ggf. menschengroßes - Objekt eine Zeitspanne benötigt, die 1 bis 2 Größenordnungen über denen der heutigen Laborsysteme liegt.

Insbesondere einem lebenden Patienten ist eine sehr lange Verweildauer in einem MPI-Scanner wohl kaum zumutbar. Überdies sind lange Messzeiten schon aus Gesichtspunkten der Nutzungsökonomie des MPI-Scanners von Nachteil.

Man kann zur Abhilfe die Unterabtastung des FOV erwägen, um den Scanvorgang zu beschleunigen. Dies kann wiederum Anlass zu Artefakten bei der MPI-Bildgebung geben, da diese mit steigendem Grad der Unterabtastung auch stärker auf Dateninterpolation angewiesen sein wird. Als Grad der Unterabtastung wird hier die Anzahl der Voxel des FOV, denen unmittelbar aus dem MPI-Scanvorgang kein MPI-Messwert zugewiesen werden kann, dividiert durch die Anzahl aller Voxel des FOV definiert.

Es ist die Aufgabe der Erfindung, ein MPI-Verfahren zur tomographischen Bildgebung mit der Möglichkeit der Unterabtastung des FOV vorzuschlagen, das eine Artefakte vermeidende Interpolation von gemessenen Partikelkonzentrationen umfasst. Dabei soll vollständig auf feldgenerierende Spulen in axialer Richtung verzichtet und durch einen kontinuierlichen Patiententischvorschub ein axial unbeschränktes Field-of-View ermöglicht werden.

Die Aufgabe wird gelöst durch ein Verfahren zur tomographischen Bildgebung eines Objekts mit im Objektinnern verteilten magnetischen Partikeln mit vorbekannter magnetischer Wirksamkeit umfassend die Schritte

- Erzeugen eines Selektionsmagnetfeldes mit einem vorbestimmten Magnetfeldgradienten in wenigstens einem feldfreien Punkt (FFP) in einer vorbestimmten Scanebene,

- Erzeugen eines zeitabhängigen, periodischen Anregungsmagnetfeldes mit einer vorbestimmten Maximalfrequenz,

- Repetierendes Verschieben des wenigstens einen FFP entlang einer vorbestimmten geschlossenen Trajektorie mit einer vorbekannten Repetitionszeit in der Scanebene,

- Hindurchbewegen des Objekts durch die Scanebene entlang einer vorbestimmten Vorschubrichtung mit einer vorbestimmten Vorschubgeschwindigkeit,

- Detektieren der Änderung des Magnetisierungszustandes der magnetischen Partikel in den von dem wenigstens einen FFP durchlaufenen Orten im Objektinnern,

- Rekonstruieren der lokalen Partikelkonzentrationen an den von dem wenigstens einen FFP durchlaufenen Orten bezüglich eines Objektkoordinatensystems,

- Interpolieren der rekonstruierten Partikelkonzentrationen auf die nicht von wenigstens einem FFP durchlaufenen Orte im Objektinnern,

- Erzeugen einer Darstellung der Partikelkonzentrationsverteilung im Objektinnern,

gekennzeichnet durch Interpolieren der an Streckenabschnittsendpunkten von Streckenabschnitten entlang der Vorschubrichtung rekonstruierten Partikelkonzentrationen auf Streckenabschnittszwischenpunkte unter Einbeziehen der Vorschubgeschwindigkeit.

Weiterhin wird die Aufgabe durch einen erfindungsgemäßen MPI-Scanner zur tomographischen Bildgebung eines Objekts mit im Objektinnern verteilten magnetischen Partikeln mit vorbekannter magnetischer Wirksamkeit gelöst. Der MPI-Scanner weist

- Mittel zum Erzeugen eines Selektionsmagnetfeldes mit einem vorbestimmten Magnetfeldgradienten in wenigstens einem feldfreien Punkt (FFP) in einer vorbestimmten Scanebene,

- Mittel zum Erzeugen eines zeitabhängigen, periodischen Anregungsmagnetfeldes mit einer vorbestimmten Maximalfrequenz,

- Mittel zum repetierenden Verschieben des wenigstens einen FFPs entlang einer vorbestimmten geschlossenen Trajektorie mit einer vorbekannten Repetitionszeit in der Scanebene,

- Mittel zum Hindurchbewegen des Objekts durch die Scanebene entlang einer vorbestimmten Vorschubrichtung mit einer vorbestimmten Vorschubgeschwindigkeit,

- Mittel zum Detektieren der Änderung des Magnetisierungszustandes der magnetischen Partikel in den von dem wenigstens einen FFP durchlaufenen Orten im Objektinnern,

- Mittel zum Rekonstruieren der lokalen Partikelkonzentrationen an den von dem wenigstens einen FFP durchlaufenen Orten bezüglich eines Objektkoordinatensystems,

- Mittel zum Interpolieren der rekonstruierten Partikelkonzentrationen auf die nicht von wenigstens einem FFP durchlaufenen Orte im Objektinnern,

- Mittel zum Erzeugen einer Darstellung der Partikelkonzentrationsverteilung im Objektinnern,

auf und ist gekennzeichnet dadurch
dass die Mittel zum Interpolieren weiterhin geeignet sind zum Interpolieren der an Streckenabschnittsendpunkten von Streckenabschnitten entlang der Vorschubrichtung rekonstruierten Partikelkonzentrationen auf Streckenabschnittszwischenpunkte unter Einbeziehen der Vorschubgeschwindigkeit.

Die Unteransprüche geben vorteilhafte Ausgestaltungen der Erfindung an.

Zur weiteren Erläuterung dienen zudem die folgenden Figuren. Dabei zeigt:

Fig. 1 a)     den Magnetisierungsverlauf M(x) eines super-paramagnetischen Eisenoxidpartikels in der Umgebung eines FFP bei x = 0, der einen vorbestimmten Magnetfeldgradienten G aufweist sowie Fig. 1 b) die Ableitung dM/dx;

Fig. 2 a)     eine zweidimensionale Lissajous-Trajektorie mit Kreuzungspunkten in der Scanebene und Fig. 2 b) die zu Fig. 2 a) gehörige expandierte FFP-Trajektorie, entlang der Messdaten im Objektkoordinatensystem erfasst werden.

Bei Goodwill et al. wird beschrieben, dass ein Objekt auf einem Tisch liegend durch eine Scanebene mit einer FFL bewegt werden kann. Goodwill et al. nutzt dabei jedoch nicht die Vorteile der Rohdatenakquisition mit einem FFP, sondern nur das Summensignal der FFL. Die entscheidende Verbesserung des hier vorgeschlagenen Verfahrens liegt nun in der Interpolation geeigneter Punkte der Messtrajektorie in axialer Richtung. Dieses Prinzip kann sowohl für die FFP-basierte als auch für die FFL-basierte Trajektorie genutzt werden.

Sowohl die Frequenz, mit der der FFP bzw. die FFL in der Scanebene bewegt wird, als auch die Vorschubgeschwindigkeit des Tisches bestimmen dabei den Grad der Unterabtastung. Denn ist die Frequenz gering und die Vorschubgeschwindigkeit hoch, dann bewegt sich der FFP bzw. die FFL durch das dreidimensionale, auf Objektkoordinaten bezogene FOV und überstreicht dabei nur noch einen Anteil aller Raumpunkte - oder Voxel - des FOV. Rotiert beispielsweise die FFL mit vorbestimmter Frequenz um ihren Mittelpunkt während der Tisch verschoben wird, dann beschreibt sie in Objektkoordinaten eine Schraubenfläche. Offensichtlich werden Voxel außerhalb der Schraubenfläche zu keiner Zeit von der FFL durchquert, und steigert man die Vorschubgeschwindigkeit, so nimmt die Anzahl dieser Voxel, und damit der Grad der Unterabtastung, zu.

Der elektrotechnische Aufwand zur Erzeugung einer MPI-Messung mit FFL ist aufwendiger als der mit einem FFP.

Es ist daher eine bevorzugte Ausgestaltung der Erfindung, anstelle einer FFL nur einen einzelnen FFP zu erzeugen und in einer zweidimensionalen Scanebene zu verschieben. In diesem Fall kann aus den MPI-Messwerten aus dem FFP jederzeit unmittelbar auf die lokale Partikelkonzentration in der Umgebung des momentanen Aufenthaltsortes des FFP geschlossen werden.

Beim Bewegen des Objekts durch die Scanebene verläuft der FFP auf einer womöglich komplizierten Bahnkurve im FOV. Die Messdaten werden beispielsweise und bevorzugt während der Dauer der MPI-Messung auf kartesischen Objektkoordinaten erfasst, die selbst vorbekannte Funktionen $x(t)$, $y(t)$, $z(t)$ des Messzeitpunktes $t$ sind.

Beispielsweise und bevorzugt beschreiben $x(t)$ und $y(t)$ eine in der Scanebene geschlossene Trajektorie mit einer Mehrzahl von Kreuzungspunkten. Hierbei kann es sich um die eingangs genannten zweidimensionalen Lissajous-Trajektorien handeln.

Vorzugsweise ist die Vorschubgeschwindigkeit $v_T$ entlang der z-Achse während der MPI-Messung wenigstens in einer vorbestimmten Untersuchungsregion des Objekts konstant, d.h. dort gilt $z(t) = z_0 + v_T * t$ mit einem vorbekannten, konstanten Offset $z_0$.

Die Gesamtheit aller vom FFP durchlaufenen Raumpunkte $(x(t), y(t), z(t))$ beschreibt die dreidimensionale FFP-Trajektorie im FOV. Sie weist keine Schnittpunkte mit sich selbst auf und durchquert womöglich nicht jeden Voxel des FOV.

Während der Dauer einer Repetitionszeit $T_R$ der zweidimensionalen, geschlossenen Trajektorie legt der FFP im FOV entlang der z-Achse eine Vorschubstrecke $v_T * T_R$ zurück, die als "Expansion" der zweidimensionalen auf die dreidimensionale FFP-Trajektorie bezeichnet werden soll.

Für die Expansion gelten folgende Aussagen:

1. Die Expansion nimmt mit der Vorschubgeschwindigkeit, mit der das Objekt durch die Scanebene bewegt wird, zu.
2. Die Expansion nimmt mit der Repetitionszeit zu, d.h. wenn die maximale Frequenz des Anregungsfeldes bei im Wesentlichen gleich bleibendem Frequenzverhältnis $f_y / f_x$ und gleich bleibender Bahnlänge der Trajektorie verringert wird.
3. Beträgt die Expansion maximal die Kantenlänge $\Delta x$ der Voxel des FOV, dann kommt es zu keiner Unterabtastung durch den Vorschub.
4. Beträgt die Expansion $p * \Delta x$ mit p größer als Eins, dann tritt durch den Vorschub bedingte Unterabtastung des FOV auf, wobei der Grad der Unterabtastung etwa $(p - 1) / p$ beträgt.

Zu Aussage 3 ist anzumerken, dass es innerhalb einer Schicht des FOV durchaus Voxel geben kann, die nicht vom FFP durchlaufen werden. Diese Unterabtastung innerhalb der Scanebene kann auch auftreten, wenn gar kein Vorschub des Objekts durch die Scanebene erfolgt. Die Ursache liegt in der Wahl der zweidimensionalen FFP-Trajektorie oder der Diskretisierung, die womöglich lückenhaft abtastet. Die bereits erwähnte US 2012/153949 A1 beschäftigt sich mit Verbesserungsmöglichkeiten zu dieser Frage, sodass der interessierte Leser im Speziellen hierauf verwiesen wird. Im Nachfolgenden wird diese Verbesserungsmöglichkeiten nicht berücksichtigt. Im Kontext der vorliegenden Beschreibung soll allein die Unterabtastung durch Vorschub betrachtet werden.

Der Grundgedanke der Erfindung ist nun, dass bei Auftreten der vorschubbedingten Unterabtastung nicht alle Voxel des FOV, für die keine MPI-Messwerte erfasst werden können, gleichermaßen problematisch bei der anschließenden Dateninterpolation sind. Vielmehr gibt es Voxel, auf denen sich auch bei einem hohen Grad der Unterabtastung immer noch robuste Schätzwerte aus den rekonstruierten Partikelkonzentrationen entlang der dreidimensionalen FFP Trajektorie errechnen lassen. Erfindungsgemäß liegen diese auf Streckenabschnitten entlang der Vorschubrichtung zwischen je zwei Streckenabschnittsendpunkten, zu denen gemessene Partikelkonzentrationen vorliegen.

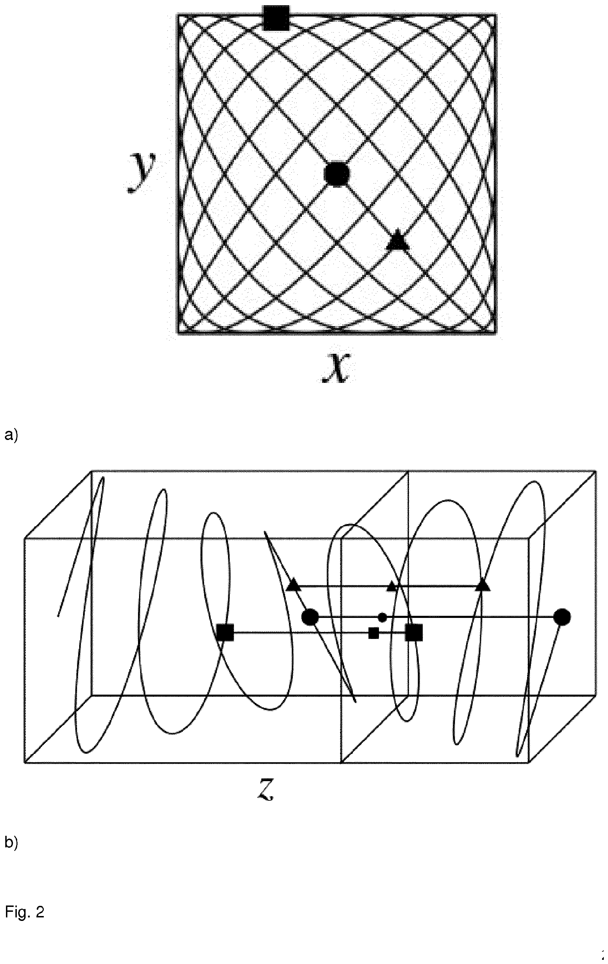

In Fig. 2 a) ist eine in der Scanebene geschlossene FFP-Trajektorie mit Kreuzungspunkten gezeichnet. Drei willkürlich gewählte Kreuzungspunkte sind markiert mit einem Kreis, einem Dreieck und einem Quadrat. Infolge der Relativverschiebung von FFP und Objekt entlang der z-Achse entsteht die dreidimensionale - auch: expandierte - FFP-Trajektorie im auf Objektkoordinaten bezogenen FOV wie gezeichnet in Fig. 2 b). Ebendort ist genau ein Durchlauf der FFP-Trajektorie mit Rückkehr in den Ausgangspunkt in der Scanebene gezeichnet, d.h. die Expansion entspricht gerade der dargestellten Breite der Fig. 2 b).

Die expandierte FFP-Trajektorie schneidet sich nicht selbst, doch die zuvor markierten Kreuzungspunkte in der Scanebene definieren nun Streckenabschnitte im Objektkoordinatensystem, die exakt entlang der z-Achse verlaufen. An beiden Endpunkten dieser Streckenabschnitte liegen jeweils aus der MPI-Messung rekonstruierte Partikelkonzentrationen vor, so dass die robuste Interpolation auf die Streckenabschnittszwischenpunkte jedenfalls dann möglich ist, wenn die Länge eines Streckenabschnitts die Breite der Punktspreizfunktion, $\Delta$x, nicht übersteigt.

Erfindungsgemäß werden Streckenabschnitte, auf denen eine robuste Interpolation möglich ist, dadurch bestimmt, dass die vorbekannten Zeitpunkte, zu denen der FFP die ebenfalls vorbekannten Kreuzungspunkte durchläuft, unter Einbeziehung der Vorschubgeschwindigkeit in Streckenlängen umgerechnet und geprüft werden, ob diese kleiner als $\Delta$x sind. Vorzugsweise ist die Vorschubgeschwindigkeit während der Messung konstant und bekannt. In diesem Fall stehen die robust interpolierbaren Voxel des FOV bereits vor der Messung fest. Doch auch wenn die Vorschubgeschwindigkeit während der Messung variieren sollte, kann die Erfindung genutzt werden, vorausgesetzt, die Vorschubgeschwindigkeit wird zusammen mit den Messdaten kontinuierlich aufgezeichnet oder sie ist als Funktion der Zeit vorbekannt.

Die Expansion der FFP-Trajektorie kann im Prinzip über das Frequenzverhältnis bzw. die Repetitionszeit der Trajektorie gesteuert werden, wird aber vorzugsweise durch die Wahl der Vorschubgeschwindigkeit kontrolliert. Eine Kontrolle mittels der Repetitionszeit, in dem die Anregungsfeldfrequenzen variiert werden, hat aber das Verschieben der Kreuzungspunkte zur Folge und erschwert insofern die Auswertung für den Fall, dass die Kreuzungspunkte nicht dynamisch - während der Datenakquisition - ermittelt, sondern a-priori berechnet und in Tabellen vorgehalten werden.

Es ist anhand von Fig. 2 b) anschaulich, dass auch für den Fall, dass die Expansion ein Mehrfaches von $\Delta$x beträgt, noch Streckenabschnitte zwischen den Kreuzungspunkten existieren können, die kürzer als $\Delta$x sind und insofern die robuste Interpolation auf die Streckenabschnittszwischenpunkte gestatten.

Betrachtet man insbesondere und beispielhaft den Fall, dass die Expansion 2 * $\Delta$x beträgt, so bedeutet dies zunächst, dass der FFP während eines einzelnen Durchlaufs der zweidimensionalen, geschlossenen Trajektorie in der Scanebene, d.h. während der Repetitionszeit $T_R$, im dreidimensionalen FOV einen Bereich umfassend zwei Schichten von Voxeln der Kantenlänge $\Delta$x durchquert. Etwa die Hälfte dieser Voxel wird dabei aber vom FFP nicht durchlaufen, d.h. der Grad der Unterabtastung durch den Vorschub liegt bei ungefähr 50 %.

Gleichwohl ist immer noch etwa die Hälfte aller Streckenabschnitte zwischen den Kreuzungspunkten kürzer als $\Delta$x, und die Streckenabschnittszwischenpunkte dieser Streckenabschnitte sind robust interpolierbar. Dies erlaubt bereits eine Teilinterpolation beider Schichten. Bedenkt man weiterhin, dass die Trajektorie repetierend durchlaufen wird und somit vorlaufende und nachlaufende MPI-Messwerte vorliegen, kann man noch weitere Voxel robust interpolieren. Denn jede geschlossene FFP-Trajektorie kann in zwei gleich lange, an Kreuzungspunkten beginnende und endende Halbtrajektorien zerlegt und zeitlich vertauscht wieder zusammengefügt werden. Anders gesagt, man kann die zweite Hälfte einer FFP-Trajektorie mit der ersten Hälfte der nachfolgenden FFP-Trajektorie verbinden und für diese neue Trajektorie genauso Kreuzungspunkte und Verbindungsstrecken zur Interpolation heranziehen.

Um die Interpolation auf alle übrigen, ursprünglich nicht vom FFP durchlaufenen Voxel bzw. Raumpunkte des FOV, abzuschließen, kann man unter Verwendung der Ergebnisse der vorrangig interpolierten Streckenabschnitte die verbliebenen Datenlücken mit einem geeigneten Algorithmus nach dem Stand der Technik, beispielsweise aus der CT-Bildrekonstruktion, schließen. Dabei ist es besonders vorteilhaft, dass die interpolierten Streckenabschnitte entlang der z-Achse verteilt vorliegen, d.h. man hat in jeder z-Ebene Bereiche mit relativ zuverlässigen Daten, entweder gemessen oder robust interpoliert, aus dem erfindungsgemäßen Verfahren zur Verfügung.

Die vorgenannte Argumentation gilt im Prinzip genauso, wenn die Expansion ein beliebiges Vielfaches von $\Delta$x beträgt. Allerdings werden die robust interpolierbaren Streckenabschnitte zwischen den Kreuzungspunkte mit wachsender Expansion immer seltener, und die zuverlässigen Bereiche in jeder z-Ebene immer kleiner. Irgendwann dominieren Artefakte der Interpolation das gewonnene Bild.

Es soll aber darauf hingewiesen sein, dass die Punktspreizfunktion aus Fig. 1 b) nicht abrupt bei x = $\pm$ $\Delta$x/2 auf Null abfällt, sondern eine größere Reichweite besitzt. Es ist also möglich, sich beim Ausführen der Erfindung die Frage zu stellen, ob man auch Streckenabschnitte mit einer Länge größer als $\Delta$x zur vorrangigen Interpolation heranziehen will. Wie so oft ist hier eine Abwägung zwischen dem notwendigen Grad an Unterabtastung und Artefakt-Toleranz in den erzeugten Bildern vorzunehmen.

Derzeit wird davon ausgegangen, dass die Expansion der FFP-Trajektorie ohne größere Nachteile für die MPI-Bildgebung zwischen $\Delta$x und 3 * $\Delta$x betragen kann. Eine Expansion bis hin zu 5 * $\Delta$x könnte unter günstigen Umständen noch zu brauchbaren Resultaten führen. Der Grad der Unterabtastung liegt hier aber bei etwa 80 %, und erhebliche Artefakte sind dann zu erwarten.

Am nachfolgenden Beispiel eines medizinischen MPI-Scanners, dessen FOV den gesamten Körper eines erwachsenen Patienten enthalten kann, wird der Vorteil der Erfindung verdeutlicht.

Ein medizinischer MPI-Scanner weist beispielsweise einen verschiebbaren Tisch zur Aufnahme eines liegenden Patienten, der mittels eines Elektromotors den Patienten der Länge nach durch eine Spulenanordnung bewegen kann.

D.h. in allgemeiner Form werden Mittel zum Hindurchbewegen des Objektes durch die Scanebene entlang einer vorbestimmten Vorschubrichtung bereitgestellt.

Die Spulen erzeugen Selektions- und Anregungsfeld derart, dass ein FFP in einer Scanebene senkrecht zur Tischvorschubrichtung erzeugt und verschoben werden kann. D.h. in allgemeiner Form werden Mittel zum Erzeugen eines Selektionsmagnetfeldes mit einem vorbestimmten Magnetfeldgradienten in wenigstens einem feldfreien Punkt (FFP) in einer vorbestimmten Scanebene, und Mittel zum Erzeugen eines zeitabhängigen, periodischen Anregungsmagnetfeldes mit einer vorbestimmten Maximalfrequenz, und Mittel zum repetierenden Verschieben des wenigstens einen FFPs entlang einer vorbestimmten geschlossenen Trajektorie mit einer vorbekannten Repetitionszeit in der Scanebene, bereitgestellt.

Die Scanebene beinhaltet ein FOV mit einem Durchmesser von 0,5 m. Zum Einrichten eines Magnetfeldgradienten des Betrages G = 1 T/m im FFP muss innerhalb des FOV eine Magnetfeldamplitude von etwa 0,25 T erzeugt werden.

Der FFP verläuft beispielsweise auf einer Lissajous-Trajektorie in der Scanebene mit einem Frequenzverhältnis nach Gleichung (2).

Der MPI-Scanner enthält weiterhin Mittel zum Detektieren der Änderung des Magnetisierungszustandes der magnetischen Partikel in den von dem wenigstens einen FFP durchlaufenen Orten im Objektinnern. Beispielsweise können diese Mittel zum Detektieren als Detektionsspulen oder als Magnetfeldsensoren ausgebildet sein.

Weiterhin weist der MPI-Scanner auch Mittel zum Rekonstruieren der lokalen Partikelkonzentrationen an den von dem wenigstens einen FFP durchlaufenen Orten bezüglich eines Objektkoordinatensystems, auf. Diese Mittel zum Rekonstruieren können beispielsweise durch eine hardware-programmierte oder programmtechnisch eingerichtete Rechnereinheit oder eine Kombination hieraus bereitgestellt werden.

Weiterhin weist der MPI-Scanner auch Mittel zum Interpolieren der rekonstruierten Partikelkonzentrationen auf die nicht von wenigstens einem FFP durchlaufenen Orte im Objektinnern auf. Die Mittel zum Interpolieren sind weiterhin geeignet zum Interpolieren der an Streckenabschnittsendpunkten von Streckenabschnitten entlang der Vorschubrichtung rekonstruierten Partikelkonzentrationen auf Streckenabschnittszwischenpunkte unter Einbeziehen der Vorschubgeschwindigkeit. Diese Mittel zum Interpolieren können beispielsweise auch durch einen hardware-programmierte oder programmtechnisch eingerichtete Rechnereinheit oder eine Kombination hieraus bereitgestellt werden, wobei hier anzumerken ist, dass die hardware-programmierte oder programmtechnisch eingerichtete Rechnereinheit zugleich auch andere Schritte des Verfahrens unterstützen kann.

Darüber hinaus weist der MPI-Scanner auch Mittel zum Erzeugen einer Darstellung der Partikelkonzentrationsverteilung im Objektinnern auf. Beispielsweise kann mittels einer Anzeigeeinheit, wie .z.B. ein Bildschirm oder einem Projektor die Partikelkonzentrationsverteilung im Objektinnern geeignet, z.B. farblich codiert, visualisiert werden.

Wenn die dem Patienten vor der MPI-Messung verabreichten magnetischen Partikel eine magnetische Wirksamkeit von $X_S = 3 * 10^{-3}$ T besitzen, beträgt die Breite der Punktspreizfunktion im FFP $\Delta x = 3$ mm, und für eine ausreichend dichte Abtastung der Scanebene werden etwa

$$Nd = 0{,}5 \text{ m} / 3 \text{ mm} = 167 \tag{3}$$

Kreuzungspunkte der Lissajous-Trajektorie entlang jedes Durchmessers benötigt.

Die maximale Anregungsfeldfrequenz, hier $f_x$, wird exemplarisch zu 100 Hz eingerichtet, um periphere Nervenstimulation sicher zu vermeiden. Die Repetitionszeit der Trajektorie ist dann per Definition

$$T_R = N_d / f_y = (N_d + 1) / f_x = 168 / 100 \text{ Hz} = 1{,}68 \text{ s} . \tag{4}$$

Diese Abschätzung liegt Größenordnungen über den Repetitionszeiten von FFP-Trajektorien in den bis heute realisierten MPI-Scannern.

Grundsätzlich ist für einen MPI-Scanner mit einer Scanebene, durch die ein Mensch hindurchbewegt werden soll, vorzusehen, dass der wenigstens eine feldfreie Punkt entlang einer geschlossenen Trajektorie mit einer Repetitionszeit größer als 500 Millisekunden, bevorzugt größer als eine Sekunde verschoben wird.

Ohne vorschubbedingte Unterabtastung wäre ein Ganzkörper-Scan des Patienten mit einer Körperlänge von z.B. 1,80 m mit einer Vorschubgeschwindigkeit von

$$v_T = 3 \text{ mm} / 1{,}68 \text{ s} = 1{,}79 \text{ mm/s} \tag{5}$$

vorzunehmen und würde insgesamt 1008 s = 16,8 min beanspruchen. Mit Hilfe der Erfindung lässt sich diese Zeit nun effektiv halbieren oder sogar dritteln, ohne dass dadurch übermäßig Artefakte in den MPI-Bildern erzeugt werden.

In einer bevorzugten Ausgestaltung ist die Maximalfrequenz des Anregungsfeldes vorbestimmt, und der Grad der Unterabtastung wird allein durch die Wahl der Vorschubgeschwindigkeit durch den Nutzer festgelegt. Diese ist erfindungsgemäß zu

$$v_T = p * X_S / (G * T_R) \qquad\qquad (6)$$

einzurichten, wobei p eine Zahl größer als Eins und kleiner als Fünf ist. Vorzugsweise ist p größer als Eins und höchstens Drei.

Es soll hier betont werden, dass es eine bevorzugte Ausgestaltung der Erfindung ist, bei einem humanen Ganzkörperscanner die maximale Anregungsfeldfrequenz, d.h. den höchstfrequenten Anteil des periodischen Anregungsfeldes, bei einer Frequenz unter 1000 Hz, bevorzugt unter 200 Hz, besonders bevorzugt um 100 Hz festzulegen.

Diese Festlegung ist für lebende Menschen physiologisch motiviert und gibt Anlass zur Unterabtastung des FOV und zur Anwendung der Erfindung; sie ist aber nicht zwingend. Einen Anlass zur Unterabtastung kann es auch aus anderen Gründen geben, etwa wenn man ein - durchaus unbelebtes - Objekt nur mäßiger magnetischer Induktion aussetzen will oder womöglich mit sehr hoher Geschwindigkeit durch die Scanebene bewegen möchte, z.B. auf einem Fließband.

Der Vollständigkeit halber sei angemerkt, dass das Hindurchbewegen des Objekts durch die Scanebene offensichtlich alternativ realisiert werden kann, indem eine bewegliche Spulenanordnung, die alle benötigten Magnetfelder erzeugt und so eine Scanebene einrichtet, derart am ruhenden Objekt vorbeigeführt wird, dass die Scanebene durch das Objekt hindurch bewegt wird. Beide Varianten sind im Sinne der Erfindung als äquivalent anzusehen.

Ohne weiteres können Aspekte der Erfindung auch in Software und / oder programmtechnisch eingerichteten Rechnereinrichtungen verkörpert sein.


**Patentansprüche**

1.  Verfahren zur tomographischen Bildgebung eines Objekts mit im Objektinnern verteilten magnetischen Partikeln mit vorbekannter magnetischer Wirksamkeit umfassend die Schritte

    • Erzeugen eines Selektionsmagnetfeldes mit einem vorbestimmten Magnetfeldgradienten in wenigstens einem feldfreien Punkt (FFP) in einer vorbestimmten Scanebene,
    • Erzeugen eines zeitabhängigen, periodischen Anregungsmagnetfeldes mit einer vorbestimmten Maximalfrequenz,
    • Repetierendes Verschieben des wenigstens einen FFP entlang einer vorbestimmten geschlossenen Trajektorie mit einer vorbekannten Repetitionszeit in der Scanebene,
    • Hindurchbewegen des Objekts durch die Scanebene entlang einer vorbestimmten Vorschubrichtung mit einer vorbestimmten Vorschubgeschwindigkeit,
    • Detektieren der Änderung des Magnetisierungszustandes der magnetischen Partikel in den von dem wenigstens einen FFP durchlaufenen Orten im Objektinnern,
    • Rekonstruieren der lokalen Partikelkonzentrationen an den von dem wenigstens einen FFP durchlaufenen Orten bezüglich eines Objektkoordinatensystems,
    • Interpolieren der rekonstruierten Partikelkonzentrationen auf die nicht von wenigstens einem FFP durchlaufenen Orte im Objektinnern,
    • Erzeugen einer Darstellung der Partikelkonzentrationsverteilung im Objektinnern,

    **gekennzeichnet durch**
    Interpolieren der an Streckenabschnittsendpunkten von Streckenabschnitten entlang der Vorschubrichtung rekonstruierten Partikelkonzentrationen auf Streckenabschnittszwischenpunkte unter Einbeziehen der Vorschubgeschwindigkeit.

2.  Verfahren nach Anspruch 1, **dadurch gekennzeichnet, dass** die Maximalfrequenz des periodischen Anregungsmagnetfeldes kleiner als 1000 Hz, bevorzugt kleiner als 200 Hz, besonders bevorzugt kleiner als 100 Hz vorbestimmt wird.

3. Verfahren nach einem der vorangehenden Ansprüche, **dadurch gekennzeichnet, dass** genau ein FFP erzeugt und entlang einer geschlossenen Trajektorie in der Scanebene repetierend verschoben wird, wobei die Trajektorie in der Scanebene Kreuzungspunkte aufweist.

4. Verfahren nach Anspruch 3, **dadurch gekennzeichnet, dass** die im Objektkoordinatensystem entlang der Vorschubrichtung beabstandeten Kreuzungspunkte der in der Scanebene geschlossenen Trajektorie als Streckenabschnittsendpunkte verwendet werden.

5. Verfahren nach einem der Ansprüche 3 oder 4, **dadurch gekennzeichnet, dass** die Repetitionszeit der Trajektorie größer als 500 Millisekunden, bevorzugt größer als 1 Sekunde eingerichtet ist.

6. Verfahren nach einem der vorangehenden Ansprüche, **dadurch gekennzeichnet, dass** die Repetitionszeit , $T_R$, der geschlossenen Trajektorie in der Scanebene und der vorbestimmte Magnetfeldgradient, G, in dem wenigsten einen FFP und die magnetische Wirksamkeit, $X_S$, der magnetischen Partikel zum Vorbestimmen der maximalen Vorschubgeschwindigkeit verwendet werden.

7. Verfahren nach Anspruch 6, **dadurch gekennzeichnet, dass** die maximale Vorschubgeschwindigkeit $v_T$ zu $v_T = p * X_S / (G * T_R)$ vorbestimmt wird, wobei p eine Zahl größer als Eins und kleiner als Fünf, bevorzugt höchstens Drei, ist.

8. Verfahren nach einem der vorangehenden Ansprüche, **dadurch gekennzeichnet, dass** die Vorschubgeschwindigkeit während des Detektierens der Änderung des Magnetisierungszustandes der magnetischen Partikel in wenigstens einer vorbestimmten Untersuchungsregion des Objekts konstant gehalten wird.

9. MPI-Scanner zur tomographischen Bildgebung eines Objekts mit im Objektinnern verteilten magnetischen Partikeln mit vorbekannter magnetischer Wirksamkeit aufweisend

   • Mittel zum Erzeugen eines Selektionsmagnetfeldes mit einem vorbestimmten Magnetfeldgradienten in wenigstens einem feldfreien Punkt (FFP) in einer vorbestimmten Scanebene,
   • Mittel zum Erzeugen eines zeitabhängigen, periodischen Anregungsmagnetfeldes mit einer vorbestimmten Maximalfrequenz,
   • Mittel zum repetierenden Verschieben des wenigstens einen $FFP_S$ entlang einer vorbestimmten geschlossenen Trajektorie mit einer vorbekannten Repetitionszeit in der Scanebene,
   • Mittel zum Hindurchbewegen des Objekts durch die Scanebene entlang einer vorbestimmten Vorschubrichtung mit einer vorbestimmten Vorschubgeschwindigkeit,
   • Mittel zum Detektieren der Änderung des Magnetisierungszustandes der magnetischen Partikel in den von dem wenigstens einen FFP durchlaufenen Orten im Objektinnern,
   • Mittel zum Rekonstruieren der lokalen Partikelkonzentrationen an den von dem wenigstens einen FFP durchlaufenen Orten bezüglich eines Objektkoordinatensystems,
   • Mittel zum Interpolieren der rekonstruierten Partikelkonzentrationen auf die nicht von wenigstens einem FFP durchlaufenen Orte im Objektinnern,
   • Mittel zum Erzeugen einer Darstellung der Partikelkonzentrationsverteilung im Objektinnern,

   **gekennzeichnet dadurch**
   **dass** die Mittel zum Interpolieren weiterhin geeignet sind zum Interpolieren der an Streckenabschnittsendpunkten von Streckenabschnitten entlang der Vorschubrichtung rekonstruierten Partikelkonzentrationen auf Streckenabschnittszwischenpunkte unter Einbeziehen der Vorschubgeschwindigkeit.

10. MPI-Scanner nach Anspruch 9, **dadurch gekennzeichnet, dass** die Maximalfrequenz des periodischen Anregungsmagnetfeldes kleiner als 1000 Hz, bevorzugt kleiner als 200 Hz, besonders bevorzugt kleiner als 100 Hz vorbestimmt wird.

11. MPI-Scanner nach Anspruch 9 oder 10, **dadurch gekennzeichnet, dass** genau ein FFP erzeugt und entlang einer geschlossenen Trajektorie in der Scanebene repetierend verschoben wird, wobei die Trajektorie in der Scanebene Kreuzungspunkte aufweist.

12. MPI-Scanner nach Anspruch 11, **dadurch gekennzeichnet, dass** die im Objektkoordinatensystem entlang der Vorschubrichtung beabstandeten Kreuzungspunkte der in der Scanebene geschlossenen Trajektorie als Streckenabschnittsendpunkte verwendet werden.

**13.** MPI-Scanner nach Anspruch 11 oder 12, **dadurch gekennzeichnet, dass** die Repetitionszeit der Trajektorie größer als 500 Millisekunden, bevorzugt größer als 1 Sekunde eingerichtet ist.

**14.** MPI-Scanner nach Anspruch nach einem der vorangehenden Ansprüche 9 bis 13, **dadurch gekennzeichnet, dass** die Repetitionszeit , $T_R$, der geschlossenen Trajektorie in der Scanebene und der vorbestimmte Magnetfeldgradient, G, in dem wenigsten einen FFP und die magnetische Wirksamkeit, $X_S$, der magnetischen Partikel zum Vorbestimmen der maximalen Vorschubgeschwindigkeit verwendet werden.

**15.** MPI-Scanner nach einem der vorangehenden Ansprüche 9 bis 14, **dadurch gekennzeichnet, dass** die Vorschubgeschwindigkeit während des Detektierens der Änderung des Magnetisierungszustandes der magnetischen Partikel in wenigstens einer vorbestimmten Untersuchungsregion des Objekts konstant gehalten wird.

**Claims**

**1.** Method for tomographic imaging an object having magnetic particles distributed in the interior of the object and having a pre-known magnetic effectiveness, comprising the following steps

- generating a selection field having a predetermined magnetic-field gradient in at least one field-free point (FFP) in a predetermined scan plane,
- generating a time-dependent, periodic magnetic drive field having a predetermined maximum frequency,
- repeatedly shifting the at least one FFP along a predetermined closed trajectory having a pre-known repetition time in the scan plane,
- moving the object through the scan plane along a predetermined feed direction at a predetermined feed velocity,
- detecting the change in the magnetization state of the magnetic particles at the locations, traversed by the at least one FFP, in the interior of the object,
- reconstructing the local particle concentrations at the locations, traversed by the at least one FFP, relative to an object coordinate system,
- interpolating the reconstructed particle concentrations onto the locations, not traversed by at least one FFP, in the interior of the object,
- generating a representation of the particle concentration distribution in the interior of the object,

**characterized by**
interpolating the particle concentrations, reconstructed at path section end points of path sections along the feed direction, onto path section intermediate points taking into account the feed velocity.

**2.** The method according to Claim 1, **characterized in that** the maximum frequency of the periodic magnetic drive field is predetermined to be smaller than 1000 Hz, preferably smaller than 200 Hz, particularly preferably smaller than 100 Hz.

**3.** The method according to one of the preceding claims, **characterized in that** precisely one FFP is generated and shifted repeatedly along a closed trajectory in the scan plane, the trajectory exhibiting crossing points in the scan plane.

**4.** The method according to Claim 3, **characterized in that** the crossing points, that are spaced apart in the object coordinate system along the feed direction, of the trajectory, closed in the scan plane, are used as path section end points.

**5.** The method according to one of Claims 3 or 4, **characterized in that** the repetition time of the trajectory is designed to be greater than 500 milliseconds, preferably greater than 1 second.

**6.** The method according to one of the preceding claims, **characterized in that** the repetition time $T_R$ of the closed trajectory in the scan plane and the predetermined magnetic-field gradient G in the at least one FFP and the magnetic effectiveness Xs of the magnetic particles are used for predetermining the maximum feed velocity.

**7.** The method according to Claim 6, **characterized in that** the maximum feed velocity $v_T$ is predetermined to be $v_T = p * X_S / (G * T_R)$, p being a number greater than one and smaller than five, preferably at most three.

8. The method according to one of the preceding claims, **characterized in that** the feed velocity is kept constant during the detection of the change of the magnetization state of the magnetic particles in at least one predetermined investigation region of the object.

9. An MPI scanner for tomographic imaging of an object having magnetic particles distributed in the interior of the object and having a pre-known magnetic effectiveness, comprising

> • means for generating a selection field having a predetermined magnetic-field gradient in at least one field-free point (FFP) in a predetermined scan plane,
> • means for generating a time-dependent, periodic magnetic drive field having a predetermined maximum frequency,
> • means for repeatedly shifting the at least one FFP along a predetermined closed trajectory having a pre-known repetition time in the scan plane,
> • means for moving the object through the scan plane along a predetermined feed direction at a predetermined feed velocity,
> • means for detecting the change in the magnetization state of the magnetic particles at the locations, traversed by the at least one FFP, in the interior of the object,
> • means for reconstructing the local particle concentrations at the locations, traversed by the at least one FFP, relative to an object coordinate system,
> • means for interpolating the reconstructed particle concentrations onto the locations, not traversed by at least one FFP, in the interior of the object,
> • means for generating a representation of the particle concentration distribution in the interior of the object,

**characterized in that**
the means for interpolating are further suitable for interpolating the particle concentrations, reconstructed at path section end points of path sections along the feed direction, onto path section intermediate points taking into account the feed velocity.

10. The MPI scanner according to Claim 9, **characterized in that** the maximum frequency of the periodic magnetic drive field is predetermined to be smaller than 1000 Hz, preferably smaller than 200 Hz, particularly preferably smaller than 100 Hz.

11. The MPI scanner according to Claim 9 or 10, characterized that precisely one FFP is generated and shifted repeatedly along a closed trajectory in the scan plane, the trajectory exhibiting crossing points in the scan plane.

12. The MPI scanner according to Claim 11, **characterized in that** the crossing points, that are spaced apart in the object coordinate system along the feed direction, of the trajectory, closed in the scan plane, are used as path section end points.

13. The MPI scanner according to Claim 11 or 12, **characterized in that** the repetition time of the trajectory is designed to be greater than 500 milliseconds, preferably greater than 1 second.

14. The MPI scanner according to one of the preceding Claims 9 to 13, **characterized in that** the repetition time $T_R$ of the closed trajectory in the scan plane and the predetermined magnetic-field gradient G in the at least one FFP and the magnetic effectiveness $X_S$ of the magnetic particles are used for predetermining the maximum feed velocity.

15. The MPI scanner according to one of the preceding Claims 9 to 14, **characterized in that** the feed velocity is kept constant during the detection of the change of the magnetization state of the magnetic particles in at least one predetermined investigation region of the object.

**Revendications**

1. Procédé d'imagerie tomographique d'un objet au moyen de particules magnétiques réparties à l'intérieur de l'objet et dotées d'une efficacité magnétique connue d'avance, comprenant les étapes suivantes :

> • génération d'un champ magnétique de sélection avec un gradient de champ magnétique prédéfini dans au moins un point dénué de champ (FFP) dans un plan de balayage prédéfini,

• génération d'un champ magnétique périodique d'excitation à dépendance temporelle à une fréquence maximale prédéfinie,

• décalage à répétition de l'au moins un FFP le long d'une trajectoire fermée prédéfinie avec une durée de répétition connue d'avance dans le plan de balayage,

• déplacement de l'objet à travers le plan de balayage le long d'un sens d'avance prédéfini à une vitesse d'avance prédéfinie,

• détection de la modification de l'état de magnétisation d particules magnétiques dans les endroits parcourus par l'au moins un FFP à l'intérieur de l'objet,

• rétablissement des concentrations locales de particules dans les endroits parcourus par l'au moins un FFP par rapport à un système de coordonnées de l'objet,

• interpolation des concentrations de particules rétablies sur les endroits non parcourus par au moins un FFP à l'intérieur de l'objet,

• génération d'une représentation de la répartition de concentration des particules à l'intérieur de l'objet,

**caractérisé par**

l'interpolation des concentrations de particules rétablies à des points terminaux de sections de distance de sections de distance le long du sens d'avance sur des points intermédiaires de sections de distance en intégrant la vitesse d'avance.

2. Procédé selon la revendication 1, **caractérisé en ce que** la fréquence maximale du champ magnétique périodique d'excitation est prédéfinie à moins de 1000 Hz, de préférence moins de 200 Hz, très préférentiellement moins de 100 Hz.

3. Procédé selon une des revendications précédentes, **caractérisé en ce que** précisément un FFP est généré et décalée à répétition le long d'une trajectoire fermée dans le plan de balayage, la trajectoire présentant des points de croisement dans le plan de balayage.

4. Procédé selon la revendication 3, **caractérisé en ce que** les points de croisement espacés dans le système de coordonnées de l'objet le long du sens d'avance de la trajectoire fermée dans le plan de balayage sont utilisés comme points terminaux de sections de distance.

5. Procédé selon une des revendications 3 ou 4, **caractérisé en ce que** la durée de répétition de la trajectoire est réglée de manière à être supérieure à 500 millisecondes, de préférence supérieure à 1 seconde.

6. Procédé selon une des revendications précédentes, **caractérisé en ce que** la durée de répétition $T_R$ de la trajectoire fermée dans le plan de balayage et le gradient de champ magnétique prédéfini G dans l'au moins un FFP et l'efficacité magnétique $X_s$ des particules magnétiques sont utilisés pour prédéfinir la vitesse d'avance maximale.

7. Procédé selon la revendication 6, **caractérisé en ce que** la vitesse d'avance maximale $v_T$ de $v_T = p * X_s / (G * T_R)$ sont prédéfinies, p étant un nombre supérieur à un et inférieur à 5, de préférence trois au maximum.

8. Procédé selon une des revendications précédentes, **caractérisé en ce que** la vitesse d'avance pendant la détection de la modification de l'état de magnétisation des particules magnétiques est maintenue constante dans au moins une zone d'examen prédéfinie de l'objet.

9. Scanner d'IMP pour l'imagerie tomographique d'un objet au moyen de particules magnétiques réparties à l'intérieur de l'objet et dotées d'une efficacité magnétique connue d'avance, présentant

• des moyens de génération d'un champ magnétique de sélection avec un gradient de champ magnétique prédéfini dans au moins un point dénué de champ (FFP) dans un plan de balayage prédéfini,

• des moyens de génération d'un champ magnétique périodique d'excitation à dépendance temporelle à une fréquence maximale prédéfinie,

• des moyens de décalage à répétition de l'au moins un FFP le long d'une trajectoire fermée prédéfinie avec une durée de répétition connues d'avance dans le plan de balayage,

• des moyens de déplacement de l'objet à travers le plan de balayage le long d'un sens d'avance prédéfini à une vitesse d'avance prédéfinie,

• des moyens de détection de la modification de l'état de magnétisation des particules magnétiques dans les endroits parcourus par l'au moins un FFP à l'intérieur de l'objet,

• des moyens de rétablissement des concentrations locales de particules aux endroits parcourus par l'au moins un FFP par rapport à un système de coordonnées de l'objet,

• des moyens d'interpolation des concentrations de particules rétablies sur les endroits non parcourus par au moins un FFP à l'intérieur de l'objet,

• des moyens de génération d'une représentation de la répartition de concentration des particules à l'intérieur de l'objet,

**caractérisé en ce que**
les moyens d'interpolation restent aptes à interpoler les concentrations de particules rétablies aux points terminaux de sections de distance des sections de distance le long du sens d'avance sur des points intermédiaires de sections de distance en intégrant la vitesse d'avance.

10. Scanner d'IMP selon la revendication 9, **caractérisé en ce que** la fréquence maximale du champ magnétique périodique d'excitation est prédéfinie à moins de 1000 Hz, de préférence moins de 200 Hz, très préférentiellement moins de 100 Hz.

11. Scanner d'IMP selon la revendication 9 ou 10, **caractérisé en ce que** précisément un FFP est généré et décalé à répétition le long d'une trajectoire fermée dans le plan de balayage, la trajectoire présentant des points de croisement dans le plan de balayage.

12. Scanner d'IMP selon la revendication 11, **caractérisé en ce que** les points de croisement espacés dans le système de coordonnées de l'objet dans le plan de balayage sont utilisés comme points terminaux de sections de distance.

13. Scanner d'IMP selon la revendication 11 ou 12, **caractérisé en ce que** la durée de répétition de la trajectoire est réglée de manière à être supérieure à 500 millisecondes, de préférence supérieure à 1 seconde.

14. Scanner d'IMP selon une des revendications précédentes 9 à 13, **caractérisé en ce que** la durée de répétition $T_R$ de la trajectoire fermée dans le plan de balayage et le gradient de champ magnétique prédéfini G dans l'au moins un FFP et l'efficacité magnétique $X_s$ des particules magnétiques sont utilisés pour prédéfinir la vitesse d'avance maximale.

15. Scanner d'IMP selon une des revendications précédentes 9 à 14, **caractérisé en ce que** la vitesse d'avance est maintenue constante pendant la détection de la modification de l'état de magnétisation des particules magnétiques dans au moins une zone d'examen prédéfinie de l'objet.

Fig. 1

a)

b)

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2012153949 A1

- WO 2010008478 A2

**In der Beschreibung aufgeführte Nicht-Patentliteratur**

- **BORGERT J. ; GLEICH B. ; RAHMER J. ; DAHNKE H. ; WEIZENECKER J.** Three-Dimensional Real-Time In Vivo Magnetic Particle Imaging. *Medica-Mundi,* 2009, vol. 53/2, 48-57
- **GOODWILL, P.W. ; KONKLE, J.J. ; ZHENG, B. ; SARITAS, E.U. ; CONOLLY, S.M.** Projection X-Space Magnetic Particle Imaging. *IEEE Trans. Med. Imag.,* vol. 31 (5), 2012

- **REILLY, J.P.** Magnetic field excitation of peripheral nerves and the heart: a comparison of thresholds. *Med. Bio. Engin. Comp.,* 1991, vol. 29 (6), 571-579